# EUROPEAN PATENT SPECIFICATION

(11) **EP 3 969 105 B1**
(45) Date of publication and mention of the grant of the patent: **14.10.2026**
(21) Application number: 20806059.0
(22) Date of filing: 14.05.2020
(51) Int. Cl.: A61N 1/372, A61B 5/00, G16H 40/67, H04W 12/00, H04W 12/06, H04W 12/08, H04W 12/50, G16H 10/60, H04W 84/18, A61N 1/378

(54) **AUTHORIZING PROGRAMMING OF AN IMPLANTED DEVICE USING SECOND FACTOR**
AUTORISIERUNG DER PROGRAMMIERUNG EINER IMPLANTIERTEN VORRICHTUNG UNTER VERWENDUNG EINES ZWEITEN FAKTORS
AUTORISATION DE PROGRAMMATION D'UN DISPOSITIF IMPLANTÉ À L'AIDE D'UN SECOND FACTEUR

(30) Priority: 14.05.2019 US 201962847359 P; 13.05.2020 US 202015930580
(43) Date of publication of application: 23.03.2022
(60) Divisional application: 26203763.3
(73) Proprietor: Verily Health Inc., Dallas, Texas 75019 (US)
(72) Inventor: CARUSO, Michael Andrew, South San Francisco, California 94080 (US); BILDERSEE, Adam, South San Francisco, California 94080 (US); PATEL, Nehal, South San Francisco, California 94080 (US); SALVIA, James, South San Francisco, California 94080 (US); GOVINDARAJ, Arvind, South San Francisco, California 94080 (US)
(74) Representative: Mewburn Ellis LLP
(86) International application number: PCT/US2020/032925
(87) International publication number: WO 2020/232266

(56) References cited:
- WO-A1-2018/156386
- US-A1- 2008 058 900
- US-A1- 2009 182 388
- US-A1- 2014 273 824
- US-A1- 2015 065 047
- US-A1- 2015 089 590
- US-A1- 2016 156 528
- US-A1- 2017 259 072
- US-A1- 2018 241 564
- SIG PROPRIETARY BLUETOOTH: "Bluetooth SIG Proprietary Bluetooth Core Specification v5.0", 6 December 2016 (2016-12-06), pages 1 - 2822, XP055567380, Retrieved from the Internet <URL:https://www.bluetooth.org/en-us/specification/adopted-specifications> [retrieved on 20190311]

## Description

### BACKGROUND

A growing number of patients have implantable medical devices to treat or monitor various illnesses. Implantable medical devices may have to pair with an external device, for example, for obtaining information from the implanted device or setting parameters for treatment. In existing systems, security of the implantable medical device is primarily achieved through obscurity. Although communications may be encrypted or otherwise secured, the original pairing connection typically involves no security steps, leaving the patient vulnerable to accidental or malicious manipulation of the implantable medical device.

WO 2018/156386 A1 describes systems, apparatus, methods and computer-readable storage media facilitating trusted pairing between an IMD and an external device. An IMD includes a housing configured to be implanted within a patient, a memory and circuitry within the housing and a processor that executes executable components stored in the memory. The executable components can include: a communication component configured to initiate establishing a telemetry connection with an external device in accordance with a first telemetry protocol based on reception of a request, from the external device, to establish the telemetry connection with the IMD using the first telemetry protocol; and a validation component configured to restrict establishment of the telemetry connection with the external device in accordance with the first telemetry protocol based on reception of validation information from the external device, wherein provision of the validation information is excluded from the first telemetry protocol.

### SUMMARY

Described herein are systems and methods for addressing the lack of security during pairing for a communication connection between an implantable medical device and an external device described above. Accordingly, there is provided a method for ensuring secure pairing with an implantable medical device and a system for second factor authorization according to the independent claims.

### BRIEF DESCRIPTION OF THE DRAWINGS

A further understanding of the nature and advantages of various examples may be realized by reference to the following figures.
FIG. 1 illustrates a simplified diagram of an implantable medical device system with second factor authorization, according to some embodiments.
FIG. 2 illustrates a swim diagram of communication exchanges for pairing an implantable medical device with an external device.
FIG. 3 illustrates a method of implementing second factor authorization for an implantable medical device according to some embodiments.
FIG. 4 illustrates an exemplary computer system.

Unless otherwise indicated, elements using the same indicator number are the same elements between differing figures. Some elements may include multiple of the same elements, which are indicated by a letter following the indicator number.

### DETAILED DESCRIPTION

An implantable medical device ("IMD") (also referred to herein as implanted device) may communicate with external devices for various reasons. For example, parameters may be set in the IMD or information read out and monitored from the IMD by communication with, for example, the patient's smartphone or a clinician's device such as a tablet or other computing device. It is desirable to ensure that a clinician or patient is connecting to the correct IMD (e.g., not some other nearby IMD) and that unauthorized persons are not connecting to the IMD.

Embodiments described herein include an IMD configured to pair with an external device (e.g., tablet, smartphone, or the like) via, for example, BLUETOOTH^{®} communication. The IMD may receive the pairing request and transmit a request for authorization. The external device may display the request for authorization, prompting the user to provide an authorization pulse. The charging device for the IMD may be configured to send an authorization pulse via the inductive charging coils to the IMD when the charging device is properly aligned with the IMD and, for example, the user presses an authorization button on the charging device. The IMD may receive the authorization pulse and proceed with pairing.

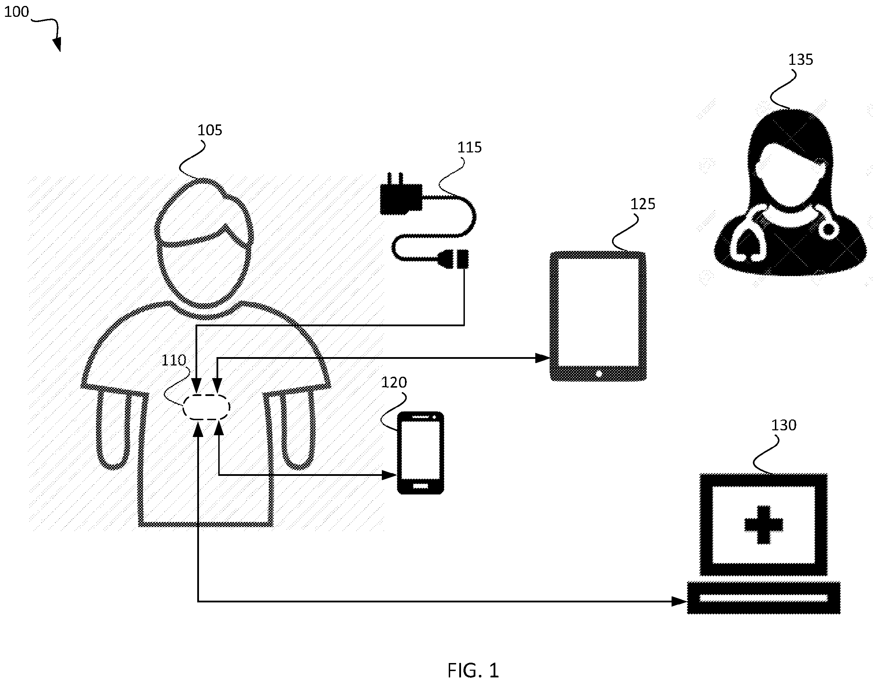

**FIG. 1** illustrates a simplified diagram 100 of components used for second factor authorization with an IMD 110. The diagram 100 includes the patient 105, the IMD 110, the charging device 115, a patient smartphone 120, a clinician tablet 125, a clinical computing device 130, and a clinician 135.

The IMD 110 may be any implantable medical device such as, for example, an implantable pulse generator ("IPG"), a pacemaker, an implantable cardiac defibrillator, and the like. The IMD 110 may be implanted into the patient 105. The IMD 110 may have at least two interfaces for communicating with external devices. The interfaces may include, for example, a short-range wireless interface, such as, for example, a BLUETOOTH^{®} antenna. Another interface may include an inductive coil, a near-field communication antenna, or any other receiver for receiving mini-range signals. The two interfaces may be different types of interfaces or the same type of interface. Interfaces may include any type of transmission including radio wave communication, magnetic communication using, for example, induction coils, near-field communication, or any other suitable type of communication transmission. Interfaces may use any type of protocol or networking including personal area networks, wide area networks, and the like. Interfaces may further use any suitable technology including ultra-high frequency, short wavelength, infrared, high-speed communication, low-speed communication, low-energy, or any other suitable technology. While patient 105 is shown with a single IMD 110, any number of IMDs may be implanted into patient 105, and the techniques described herein may be used to distinguish between and communicate with each IMD 110 individually.

The charging device 115 may be any suitable device for communicating with the IMD 110. The charging device may include, for example, an inductive coil for generating an electromagnetic field that may be received by the inductive coil in the IMD 110 for charging the IMD 110. To charge the IMD 110, the charging device 115 may be placed close to or touching the body of the patient 105 and aligned with the IMD 110 within the patient 105. The charging device may include a button used to initiate charging of the IMD 110. In some embodiments, the charging device may have an authorization button that initiates an authorization pulse using the inductive coil. For example, the authorization pulse may be a set of short pulses. Any pulsing signal may be used as long as IMD 110 is configured to recognize the pulse pattern as the authorization pulse. The pulses may form an encoded command used by the IMD 110 as an authorization. The authorization pulse contains data, including metadata in some embodiments. As described in more detail herein, the authorization pulse provides data that authorizes and instructs the IMD 110 to complete pairing with the external device (e.g., the patient smartphone 120, clinician tablet 125, clinical computing device 130, or the like). Other messages sent via the charging device 115 to IMD 110 are distinguishable from the authorization pulse. For example, an emergency turn off instruction can be transmitted to the IMD 110 using charging device 115, which is distinguishable from the authorization pulse transmitted using charging device 115.

In some embodiments, rather than a separate authorization button, the button used to initiate charging may be used to transmit the encoded command. For example, a long-hold of the button or a double click may be used to initiate the authorization pulse. Note also that while charging device 115 may be a charging device for charging the IMD 110, any device capable of communicating with the IMD 110 can be used to provide the authorization pulse. Incorporating the authorization pulse capability into the charging device 115 that charges the IMD 110 saves components, cost, and space. However, a separate device can be used to provide the authorization pulse using the inductive coil in the IMD 110 or any other antenna within IMD 110 for receiving the authorization pulse. Further, the method for initiating the authorization pulse may be any suitable component that captures the user or patient intent to send the authorization pulse so that it is unlikely that the user has accidentally triggered the authorization pulse or was tricked into performing the action. For example, long-holding a button or a locked trigger that must be unlocked before triggering would be less likely to be accidentally triggered or a user tricked into triggering. Additionally, use of the charging device that uses an inductive coil for communication is mini-range, such that it is unlikely the patient would be unaware of the authorization pulse action because of the proximity to the patient that is required to transmit an inductive pulse. These various features help ensure the patient actually intends to authorize communication with the external device such as the patient smartphone 120.

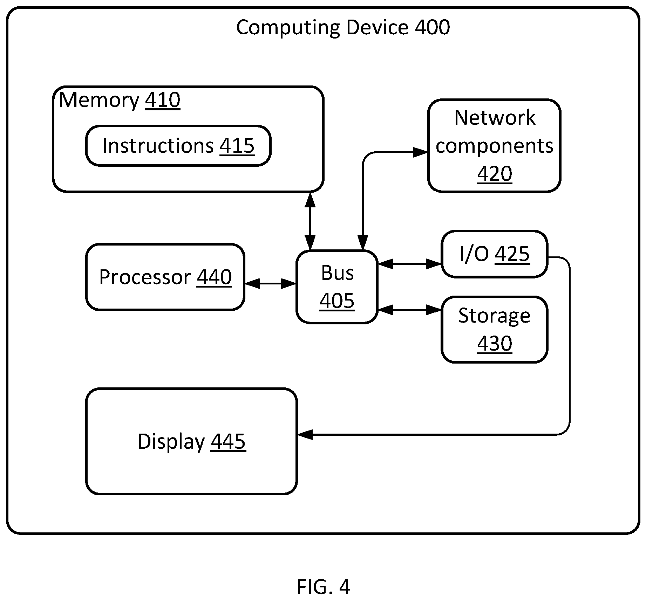

Patient smartphone 120 may be any suitable general-purpose electronic device. A general-purpose electronic device is one that performs computing tasks with the ability to store instructions on a memory device and execute those instructions with a processor (e.g., a smartphone, a tablet, a desktop computer, a laptop computer, any mobile computing device, or the like). Patient smartphone 120 may be computing device 400 of FIG. 4, for example. Patient smartphone 120 may be a computing device of the patient 105 used to communicate with the IMD 110. For example, the patient 105 may need to set a parameter of the IMD 110 or monitor a value provided by the IMD 110. Patient smartphone 120 may include at least one compatible interface for communicating with IMD 110. Communication between the IMD 110 and the patient smartphone 120 may be through wireless technology including, for example, short-wavelength radio waves, ultra-high frequency radio waves, a personal area network, a packet-based protocol, a master-slave architecture, any combination thereof, or any other suitable wireless communication technology. For example, patient smartphone 120 may include a short-range (e.g., approximately 35 feet between devices or less) wireless communication interface, such as, for example, a BLUETOOTH^{®} antenna. Patient smartphone 120 may be configured with an interface for obtaining information from or setting parameters of the IMD 110. For example, patient 105 may install an app on patient smartphone 120 that allows the patient 105 to use the patient smartphone 120 to use a graphical user interface to exchange information with the IMD 110. For example, the app may include security certificates used to interface with IMD 110. The patient smartphone 120 may be used via the interface to set parameters on the IMD 110 or obtain information from the IMD 110 (e.g., sensor data). While only one patient smartphone 120 is depicted in FIG. 1, patient 105 may have any number of computing devices that support communication with IMD 110. For example, patient 105 may have a smartphone, a tablet, a notebook computer, a desktop computer, and so forth. Each device may communicate with IMD 110 using the described techniques.

Clinician tablet 125 may be any suitable general-purpose electronic device. Clinician tablet 125 may be computing device 400 of FIG. 4, for example. Clinician tablet 125 may be a computing device used by the clinician 135 used to communicate with IMD 110. For example, the clinician 135 may be performing a service to the patient 105 and, for example, may need to set a parameter of the IMD 110 or monitor a value provided by the IMD 110. Clinician tablet 125 may include at least one compatible interface for communicating with IMD 110. Communication between the IMD 110 and the clinician tablet 125 may be through wireless technology including, for example, short-wavelength radio waves, ultra-high frequency radio waves, a personal area network, a packet-based protocol, a master-slave architecture, any combination thereof, or any other suitable wireless communication technology. For example, clinician tablet 125 may include a short-range wireless communication interface, such as, for example, a BLUETOOTH^{®} antenna. Clinician tablet 125 may be configured with an interface for obtaining information from or setting parameters of the IMD 110. For example, clinician 135 may install an app on clinician tablet 125 that allows the clinician to use the clinician tablet 125 to use a graphical user interface to exchange information with the IMD 110. For example, the app may include security certificates used to interface with IMD 110. The clinician tablet 125 may be used via the interface to set parameters on the IMD 110 or obtain information from the IMD 110 (e.g., sensor data). While only one clinician tablet 125 is depicted in FIG. 1, clinician 135 may have any number of computing devices that support communication with IMD 110. For example, clinician 135 may have a tablet, a smartphone, a notebook computer, a desktop computer, and so forth. Each device may communicate with IMD 110 using the described techniques.

Clinical computing device 130 may be any suitable general-purpose electronic device. Clinical computing device 130 may be computing device 400 of FIG. 4, for example. Hospitals and other medical facilities may have any number of clinical computing devices 130 that may be used to interface with IMD 110. Clinical computing device 130 may be a computing device used by the clinician 135 that may be performing a service to the patient 105, and the clinician 135 may use the clinical computing device 130 to communicate with IMD 110. For example, the clinician 135 may need to set a parameter of the IMD 110 or monitor a value provided by the IMD 110. Clinical computing device 130 may include at least one compatible interface for communicating with IMD 110. Communication between the IMD 110 and the clinical computing device 130 may be through wireless technology including, for example, short-wavelength radio waves, ultra-high frequency radio waves, a personal area network, a packet-based protocol, a master-slave architecture, any combination thereof, or any other suitable wireless communication technology. For example, clinical computing device 130 may include a short-range wireless communication interface, such as a BLUETOOTH^{®} antenna. Clinical computing device 130 may be configured with an interface for obtaining information from or setting parameters of the IMD 110. For example, clinician 135 may install an app on clinical computing device 130 that allows the clinician to use the clinical computing device 130 to use a graphical user interface to exchange information with the IMD 110. For example, the app may include security certificates used to interface with IMD 110. The clinical computing device 130 may be used via the interface to set parameters on the IMD 110 or obtain information from the IMD 110 (e.g., sensor data). While only one clinical computing device 130 is depicted in FIG. 1, clinician 135 may have any number of computing devices that support communication with IMD 110. Each device may communicate with IMD 110 using the described techniques.

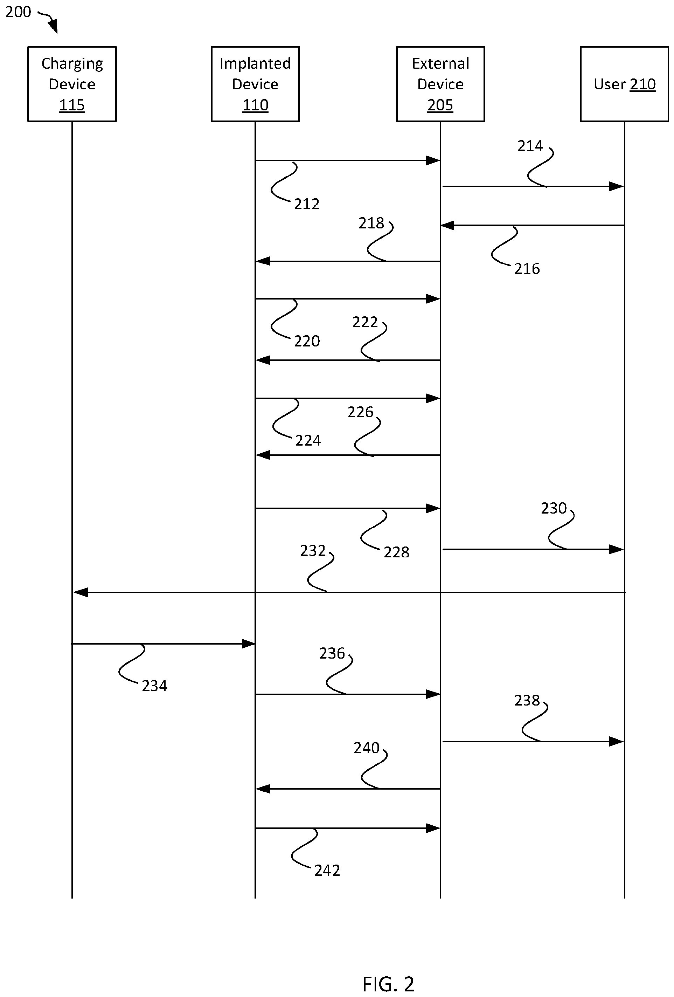

**FIG. 2** illustrates a swim diagram 200 showing message passing during pairing. Along the top of the swim diagram 200 are the components used at each stage including the charging device 115, implanted device 110, external device 205, and user 210. External device 205 may be any of patient smartphone 120, clinician tablet 125, or clinical computing device 130. User 210 may be any of patient 105 and clinician 135. In some embodiments, another authorization device may be used other than the charging device 115, in which case the charging device 115 may be replaced in the swim diagram 200 with another authorization device.

At communication arrow 212, the IMD 110 may advertise its presence using, for example, a broadcast message over, for example a short-range wireless communication network (e.g., BLUETOOTH^{®}). External device 205 may receive the advertisement and provide a user interface ("UI") prompt to the user 210. For example, the user 210 may open the installed app and look for available devices. The advertisement from IMD 110 may prompt the UI to update showing the IMD 110 as available for connection.

The user 210 may select IMD 110 from the UI, and the selection may be received at the external device 205 at communication arrow 216. At communication arrow 218 the external device 205 may initiate a communication channel with IMD 110 (e.g., BLUETOOTH^{®} low energy bond). At communication arrow 220 IMD 110 may complete the communication channel with a response. The short-range wireless communication channel (e.g., BLUETOOTH^{®}) may be insecure at this point.

At communication arrow 222, external device 205 may transmit a list of supported protocol version numbers as well as a public key. The public key may be generated from the app installed on external device 205. At communication arrow 224, IMD 110 may transmit a response to external device 205 that includes, for example, the protocol version number, an IMD certificate, an indication of whether the external device 205 is pre-authorized, and a pseudorandom value that is, for example, 32 bytes. Upon receipt of the response, external device 205 may verify the IMD certificate by checking that the IMD certificate chains back to the common trust root using, for example, the X.509-based public key infrastructure.

At communication arrow 226, the external device 205 transmits back to the IMD 110 an external device certificate and the 32 byte pseudorandom value. Upon receipt, IMD 110 may verify the external device certificate. If the external device public key is saved as trusted, then pairing is complete and no further steps are necessary. Otherwise, the IMD 110 checks that the external device certificate chains back to the common trust root using, for example, the X.509-based public key infrastructure.

At communication arrow 228 the IMD 110 sends a request for authorization to the external device 205. The external device generates a UI prompt and displays it to the user 210 as shown by communication arrow 230. The UI prompt may be a prompt that is displayed in the app that is textual and/or may include an image. For example, the UI prompt may be text that states "Provide authorization" or the like. As another example, an image of the charger (or other authorization device if the charging device 115 is not used for the authorization device) being aligned with the IMD 110 may be shown. The image may be animated, for example.

At communication arrow 232, the user 210 may access the charging device 115. The user may align the charging device 115 with the IMD 110 as if the user were going to charge the IMD. Rather than initiating charging, the user 210 may initiate an authorization pulse. For example, the user 210 may long-press a button on the charging device 115 to initiate the authorization pulse. If the charging device 115 is properly aligned with the IMD 110, the IMD 110 may receive the authorization pulse as shown by communication arrow 234. The authorization pulse may be, for example, a pulsed signal over inductive charging coils. The charging device 115 that provides the authorization pulse may provide the same authorization pulse to any IMD 110. In some embodiments, the charging device 115 may be uniquely coupled to the IMD 110 such that a unique authorization pulse is provided to IMD 110 that would not work for other IMDs. The pulsed signal may be, for example, transmission of data through patterns of an on/off signal (i.e., a digital signal). Any suitable pulsing pattern may be used as long as IMD 110 is configured to recognize the pulsing pattern. For example, IMD 110 may be configured to recognize a specific pulsing pattern as an authorization pulse in response to a request for authorization. When IMD 110 is awaiting an authorization pulse and receives the current pulsing pattern, IMD 110 will recognize the authorization pulse and proceed with pairing. The data contained in the pulsed signal may include additional data (e.g., metadata) that may be unique to a specific IMD 110 and/or charging device 115. The charging device 115 may be uniquely coupled to the IMD 110, and the metadata may be used to confirm the correct charging device 115 is communicating with the correct IMD 110, for example. The authorization pulse provides data that authorizes and instructs the IMD 110 to complete pairing with the external device (e.g., the patient smartphone 120, clinician tablet 125, clinical computing device 130, or the like). Other messages sent via the charging device 115 to IMD 110 are distinguishable from the authorization pulse. For example, an emergency turn off instruction can be transmitted to the IMD 110 using charging device 115, which is distinguishable from the authorization pulse transmitted using charging device 115.

Upon receiving the authorization pulse, IMD 110 may save the external device certificate and compute the session key to finalize pairing with the external device 205. At communication arrow 236, the IMD 110 may send an indication that pairing is complete to external device 205. Future communications may use the generated session key. The session key may be used to ensure application data exchanged between the IMD 110 and external device 205 is encrypted. At communication arrow 238, external device 205 may provide an indication to user 210 that pairing is successful via a message displayed to the user 210. In some embodiments, if the authorization pulse is not received within a threshold period of time, the authorization period may time out, and the process may have to begin again at communication arrow 212.

At communication arrows 240 and 242, external device 205 and IMD 110 may exchange application data using the session key. For example, the IMD 110 may provide data to the external device 205 including configuration setting information and sensor collection data, for example. The external device 205 may request such data and, for example, modify configuration setting information in the IMD 110.

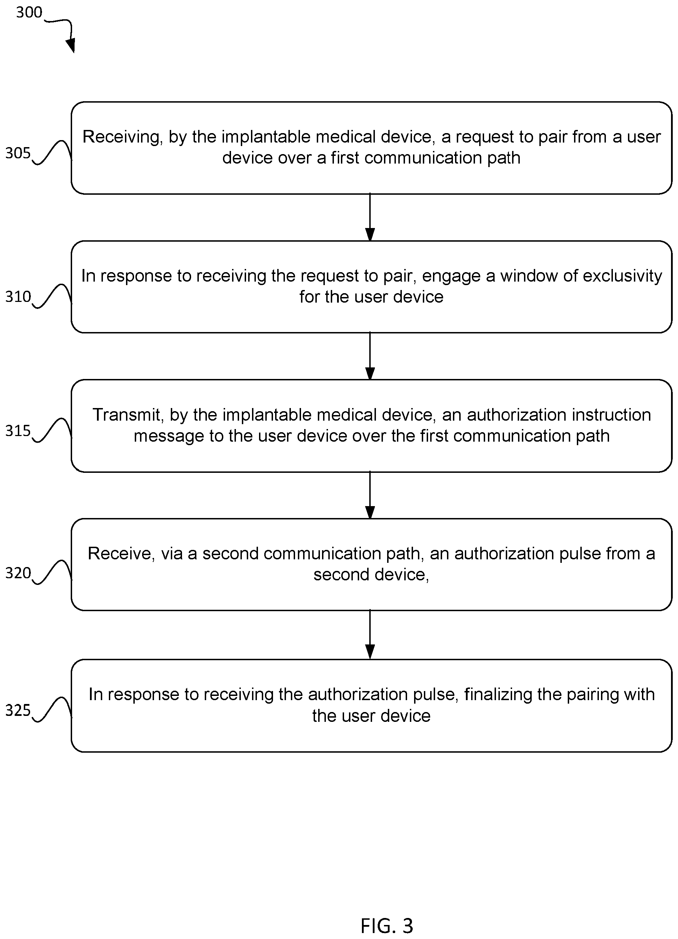

**FIG. 3** illustrates a method 300 for using second factor authorization between an IMD and an external device. Method 300 may be performed by, for example, an IMD, such as IMD 110. Method 300 begins with step 305 when the IMD receives a request to pair from a user device over a first communication path. The first communication path may use a first communication protocol. The first communication path may use a first communication interface of the IMD to send and receive signals to communicate with a communication interface of the external device. For example, the user device may be a patient device (e.g., patient smartphone 120) or a clinician device (e.g., clinician tablet 125 or clinical computer system 130) having a communication interface that may be used to communicate with the first communication interface of the IMD. The request to pair may come in response to an advertisement or broadcast sent by the IMD for communication using the first communication interface. The first communication path may be a short range wireless communication path such as, for example, BLUETOOTH^{®}. The pairing request may be, for example, a BLUETOOTH^{®} pairing request.

At step 310, in response to receiving the request to pair, the IMD may engage a window of exclusivity for the user device. The window of exclusivity may be a specific time window during which no device other than the user device that sent the request to pair to the IMD may pair with the IMD. For example, any requests to pair that the IMD receives during the window of exclusivity may be denied. In some embodiments, the IMD may stop broadcasting or advertising its presence during the window of exclusivity. The window of exclusivity may be any amount of time sufficient for a user to provide the authorization pulse such as, for example, any amount of time between thirty (30) seconds to one hundred twenty (120) seconds, though the window may be shorter than thirty seconds or longer than one hundred twenty seconds. This window of exclusivity may prevent other devices from intruding or sniping the authorization. If the window of exclusivity ends without authorization, the pairing request may be denied. If the pairing completes successfully, the window of exclusivity can be terminated in some embodiments. Once the window of exclusivity ends, the IMD may pair with other devices again. A new request to pair may then initiate to cause the IMD to engage a new window of exclusivity for the device that sent the new request to pair.

At step 315, the IMD may transmit an authorization instruction message to the user device over the first communication path. The authorization instruction message may be an indication to provide the authorization pulse. The authorization instruction message may contain digital human-comprehensible content that describes how the user may provide the authorization pulse and may include text, images, video, audio, or any combination thereof. The authorization instruction message may contain an instruction for the user device to display a message on a graphical user interface to the user to provide the authorization pulse. In some embodiments, the instruction to display the message may indicate that the user should provide the authorization pulse with the authorization device and/or may include the message to display including text, images, video, and audio. The user device may provide a prompt to the user to provide the authorization pulse. For example, the prompt may include a textual, graphical, or animated indication, such as an image of aligning the charging device with the IMD and an arrow pointing to the authorization button, to prompt the user to send the authorization pulse. Receiving this indication from the IMD may help the user know that he or she is connecting to the correct IMD. The authorization pulse may be provided by the user by, for example, aligning the charging device for the IMD with the IMD as though the user were going to initiate charging. Then, rather than initiating charging, the user may, for example, press a special authorization button or, as another example, long press the charge initiation button to initiate the authorization pulse. The authorization pulse may be a set of short pulses sent through the charging induction coils of the charging device to the charging induction coils of the IMD. The authorization pulse may include an indication that it is the authorization pulse. In some embodiments, engaging the window of exclusivity may cause the IMD to accept any pulse received over the second communication path, by a charging device, and/or by the expected receiving antenna or component as the authorization pulse. Induction communication is very close range (i.e., mini-range) (e.g., less than one (1) foot), meaning the authorization pulse may not be sent and received by the IMD without the patient likely being aware because the charging device must be, in many cases, touching the patient or within an inch at most. With, for example, a charging device, a user will typically hold the charging device against the body with only body tissue and clothing between the charging device and the IMD. Other communications can be used, such as near-field communication for transmitting the authorization pulse. However, to ensure security, the communication may be mini-range for the authorization communication protocol. Further, additional antennas increase the size of the IMD, which is ideally as small as possible since it is implanted in the patient. Since the IMD will already have induction coils and communicate with the charging device to charge the IMD, using the charging device to send the authorization pulse removes the need to increase the size of the IMD to accommodate an additional antenna.

At step 320, the IMD receives the authorization pulse from the second device (e.g., charging device) over a second communication path. The second communication path may use a different protocol than the protocol used for the first communication path, or the second communication path may use the same protocol than the protocol used for the first communication path. The second communication path may use a second communication interface of the IMD. For example, the second communication interface may be an induction coil while the first communication interface may be a radio wave transceiver. At step 325, in response finalizes the pairing with the user device. When the pairing is finalized, the user can be assured that he or she paired with the correct IMD since the authorization pulse was sent to the specific IMD. Upon finalizing pairing, the user device can communicate with the IMD over the first communication path such that general purpose components of the user device can now send messages across the communication path. For example, a configuration application on the user device may be used to transmit parameter values (e.g., configuration settings) to the IMD over the first communication path to set the parameter values within the IMD. When the IMD receives the parameter values over the first communication path, the IMD may modify the parameter values stored within the IMD to those sent from the user device.

**FIG. 4** illustrates a block diagram of an example computer system 400. Computer system 400 can be any of the described computers herein including, for example, smartphone 120, tablet 125, or machine 130. The computing device 400 can be or include, for example, a laptop computer, desktop computer, tablet, e-reader, smart phone or mobile device, smart watch, personal data assistant (PDA), or other electronic device.

The computing device 400 can include a processor 440 interfaced with other hardware via a bus 405. A memory 410, which can include any suitable tangible (and non-transitory) computer readable medium, such as RAM, ROM, EEPROM, or the like, can embody program components (e.g., instructions 415) that configure operation of the computing device 400. In some examples, the computing device 400 can include input/output ("I/O") interface components 425 (e.g., for interfacing with a display 445, keyboard, or mouse) and additional storage 430.

The computing device 400 can include network components 420. Network components 420 can represent one or more of any components that facilitate a network connection. In some examples, the network components 420 can facilitate a wireless connection and include wireless interfaces such as IEEE 802.11, BLUETOOTH^{®}, or radio interfaces for accessing cellular telephone networks (e.g., a transceiver/antenna for accessing CDMA, GSM, UMTS, or other mobile communications network). In other examples, the network components 420 can be wired and can include interfaces such as Ethernet, USB, or IEEE 1394.

Although FIG. 4 depicts a single computing device 400 with a single processor 440, the system can include any number of computing devices 400 and any number of processors 440. For example, multiple computing devices 400 or multiple processors 440 can be distributed over a wired or wireless network (e.g., a Wide Area Network, Local Area Network, or the Internet). The multiple computing devices 400 or multiple processors 440 can perform any of the steps of the present disclosure individually or in coordination with one another.

Each of the calculations or operations described herein may be performed using a computer or other processor having hardware, software, and/or firmware. The various method steps may be performed by modules, and the modules may comprise any of a wide variety of digital and/or analog data processing hardware and/or software arranged to perform the method steps described herein. The modules optionally comprising data processing hardware adapted to perform one or more of these steps by having appropriate machine programming code associated therewith, the modules for two or more steps (or portions of two or more steps) being integrated into a single processor board or separated into different processor boards in any of a wide variety of integrated and/or distributed processing architectures. These methods and systems will often employ a tangible media embodying machine-readable code with instructions for performing the method steps described above. Suitable tangible media may comprise a memory (including a volatile memory and/or a non-volatile memory), a storage media (such as a magnetic recording on a floppy disk, a hard disk, a tape, or the like; on an optical memory such as a CD, a CD-R/W, a CD-ROM, a DVD, or the like; or any other digital or analog storage media), or the like.

Different arrangements of the components depicted in the drawings or described above, as well as components and steps not shown or described are possible. Similarly, some features and sub-combinations are useful and may be employed without reference to other features and sub-combinations. Embodiments of the present disclosure have been described for illustrative and not restrictive purposes, and alternative embodiments will become apparent to readers of this patent. In certain cases, method steps or operations may be performed or executed in differing order, or operations may be added, deleted or modified. It can be appreciated that, in certain aspects of the present disclosure, a single component may be replaced by multiple components, and multiple components may be replaced by a single component, to provide an element or structure or to perform a given function or functions. Except where such substitution would not be operative to practice certain embodiments of the present disclosure, such substitution is considered within the scope of the present disclosure.

It is to be understood that the figures and descriptions of embodiments of the present disclosure have been simplified to illustrate elements that are relevant for a clear understanding of the present disclosure. Those of ordinary skill in the art will recognize, however, that these and other elements may be desirable. However, because such elements are well known in the art, and because they do not facilitate a better understanding of the present disclosure, a discussion of such elements is not provided herein. It should be appreciated that the figures are presented for illustrative purposes and not as construction drawings. Omitted details and modifications or alternative embodiments are within the purview of persons of ordinary skill in the art.

It can be appreciated that, in certain aspects of the present disclosure, a single component may be replaced by multiple components, and multiple components may be replaced by a single component, to provide an element or structure or to perform a given function or functions. Except where such substitution would not be operative to practice certain embodiments of the present disclosure, such substitution is considered within the scope of the present disclosure. A recitation of "a", "an" or "the" is intended to mean "one or more" unless specifically indicated to the contrary.

Use herein of the word "or" is intended to cover inclusive and exclusive OR conditions. In other words, A or B or C includes any or all of the following alternative combinations as appropriate for a particular usage: A alone; B alone; C alone; A and B only; A and C only; B and C only; and A and B and C.

The examples presented herein are intended to illustrate potential and specific implementations of the present disclosure. It can be appreciated that the examples are intended primarily for purposes of illustration of the present disclosure for those skilled in the art. There may be variations to these diagrams or the operations described herein without departing from the scope of the present disclosure. For instance, in certain cases, method steps or operations may be performed or executed in differing order, or operations may be added, deleted or modified.

Furthermore, whereas particular embodiments of the present disclosure have been described herein for the purpose of illustrating the present disclosure and not for the purpose of limiting the same, it will be appreciated by those of ordinary skill in the art that numerous variations of the details, materials and arrangement of elements, steps, structures, and/or parts may be made within the principle and scope of the present disclosure as long as said variations fall within the terms of the appended the claims.

## Claims

1. A method (300) for ensuring secure pairing with an implantable medical device, the method comprising:
receiving (305), by the implantable medical device, a request to pair from an external device over a first communication path;
in response to the request to pair, transmit (315), by the implantable medical device, an authorization instruction message to the external device over the first communication path;
receive (320), by the implantable medical device via a second communication path, an authorization pulse from an authorization device to authorize the request to pair, wherein the authorization device is not the external device; and
in response to receiving the authorization pulse, finalizing (325), by the implantable medical device, the pairing of the implantable medical device with the external device,
**characterized in that** the method further comprises:
in response to receiving the request to pair, enabling (310), by the implantable medical device, a window of exclusivity for the external device and denying all other requests to pair while the window of exclusivity is engaged.

2. The method of claim 1, wherein the authorization device is a charging device for the implantable medical device.

3. The method of claim 1, wherein the authorization pulse is an encoded command instructing the implantable medical device to authorize the request to pair.

4. The method of claim 1, wherein the request to pair is a request to initiate a pairing relationship between the implantable medical device and the external device using short-wavelength ultra-high frequency radio waves.

5. The method of claim 1, wherein the implantable medical device is implanted into a patient, and wherein the external device is configured with an interface for obtaining information from or setting parameters of the implantable medical device.

6. The method of claim 1, wherein engaging the window of exclusivity for the external device comprises cancelling a broadcast message, and/or
wherein engaging the window of exclusivity comprises declining all other pairing requests over a communication protocol of the first communication path.

7. The method of claim 1, wherein the authorization instruction message comprises an instruction to display a message on a graphical user interface of the external device requesting the authorization pulse, and/or
wherein the authorization instruction message comprises an instruction to display a message on a graphical user interface of the external device requesting the authorization pulse with the authorization device, and/or
wherein the authorization instruction message comprises digital human-comprehensible content describing how a user can cause the authorization device to provide the authorization pulse.

8. The method of claim 1, further comprising:
securing communication with the external device over the first communication path using an encrypted key pair.

9. The method of claim 1, wherein the second communication path is an inductive communication path, and/or
wherein the second communication path is a near-field communication path.

10. The method of claim 1, wherein the authorization pulse comprises metadata uniquely identifying the authorization device.

11. A system for second factor authorization, the system comprising an implantable medical device (110) and an authorization device, wherein:
the implantable medical device (110) comprises a short-range radio antenna, a first mini-range antenna, a processor, and a memory storing instructions that, when executed by the processor, cause the processor to:
receive a request to pair from an external device via the short-range radio antenna;
transmit a request for an authorization to the external device via the short-range radio antenna;
receive an authorization pulse from an authorization device via the first mini-range antenna to authorize the request to pair, wherein the authorization device is not the external device; and
in response to receiving the authorization pulse, complete pairing of the implantable medical device with the external device via the short-range radio antenna; and
the authorization device comprises an authorization pulse trigger and a second mini-range antenna configured to communicate with the first mini-range antenna, wherein the authorization pulse trigger causes an authorization pulse to transmit from the second mini-range antenna to the first mini-range antenna,
**characterised in that** the instructions comprise further instructions that, when executed by the processor, cause the processor to:
in response to receiving the request to pair, engage a window of exclusivity; and
deny all other requests to pair via the short-range radio antenna while the window of exclusivity is engaged.

12. The system of claim 11, wherein the first mini-range antenna is an induction coil.

13. The system of claim 11, wherein the authorization device is a charging device capable of charging a battery of the implantable medical device.

14. The system of claim 11, wherein the authorization pulse is an encoded command instructing the implantable medical device to authorize the request to pair.

15. The system of claim 11, wherein the request for the authorization comprises a message requesting the authorization pulse from the authorization device.

## Patentansprüche

1. Verfahren (300) zum Gewährleisten einer sicheren Gerätekopplung mit einer implantierbaren medizinischen Vorrichtung, wobei das Verfahren Folgendes umfasst:
Empfangen (305) einer Anfrage zur Gerätekopplung von einer externen Vorrichtung über einen ersten Kommunikationspfad durch die implantierbare medizinische Vorrichtung;
als Reaktion auf die Anfrage zur Gerätekopplung Senden (315) einer Autorisierungsbefehlsnachricht an die externe Vorrichtung über den ersten Kommunikationspfad durch die implantierbare medizinische Vorrichtung;
Empfangen (320) eines Autorisierungsimpulses von einer Autorisierungsvorrichtung durch die implantierbare medizinische Vorrichtung über einen zweiten Kommunikationspfad, um die Anfrage zur Gerätekopplung zu autorisieren, wobei die Autorisierungsvorrichtung nicht die externe Vorrichtung ist; und
als Reaktion auf das Empfangen des Autorisierungsimpulses Abschließen (325) der Gerätekopplung der implantierbaren medizinischen Vorrichtung mit der externen Vorrichtung durch die implantierbare medizinische Vorrichtung,
**dadurch gekennzeichnet, dass** das Verfahren ferner Folgendes umfasst:
als Reaktion auf das Empfangen der Anfrage zur Gerätekopplung Aktivieren (310) eines Exklusivitätsfensters für die externe Vorrichtung und Ablehnen aller anderer Anfragen zur Gerätekopplung, während das Exklusivitätsfenster aktiviert ist, durch die implantierbare medizinische Vorrichtung.

2. Verfahren nach Anspruch 1, wobei die Autorisierungsvorrichtung eine Ladevorrichtung für die implantierbare medizinische Vorrichtung ist.

3. Verfahren nach Anspruch 1, wobei der Autorisierungsimpuls ein verschlüsselter Befehl ist, der die implantierbare medizinische Vorrichtung anweist, die Anfrage zur Gerätekopplung zu autorisieren.

4. Verfahren nach Anspruch 1, wobei die Anfrage zur Gerätekopplung eine Anfrage zum Initiieren einer Gerätekopplungsbeziehung zwischen der implantierbaren medizinischen Vorrichtung und der externen Vorrichtung unter Verwendung von Funkwellen mit Ultrahochfrequenz und kurzen Wellenlängen ist.

5. Verfahren nach Anspruch 1, wobei die implantierbare medizinische Vorrichtung in einen Patienten implantiert ist, und wobei die externe Vorrichtung mit einer Schnittstelle zum Erhalten von Informationen von oder von Einstellparametern der implantierbaren medizinischen Vorrichtung ausgelegt ist.

6. Verfahren nach Anspruch 1, wobei das Aktivieren des Exklusivitätsfensters für die externe Vorrichtung das Abbrechen einer Broadcast-Sendenachricht umfasst und/oder
wobei das Aktivieren des Exklusivitätsfensters das Ablehnen aller anderer Gerätekopplungsanfragen über ein Kommunikationsprotokoll des ersten Kommunikationspfads umfasst.

7. Verfahren nach Anspruch 1, wobei die Autorisierungsbefehlsnachricht einen Befehl zum Anzeigen einer Nachricht auf einer grafischen Benutzerschnittstelle der externen Vorrichtung, die den Autorisierungsimpuls anfragt, umfasst und/oder
wobei die Autorisierungsbefehlsnachricht einen Befehl zum Anzeigen einer Nachricht auf einer grafischen Benutzeroberfläche der externen Vorrichtung, die den Autorisierungsimpuls mit der Autorisierungsvorrichtung anfragt, umfasst und/oder
wobei die Autorisierungsbefehlsnachricht digitalen von Menschen verstehbaren Inhalt umfasst, der beschreibt, wie ein Benutzer bewirken kann, dass die Autorisierungsvorrichtung den Autorisierungsimpuls bereitstellt.

8. Verfahren nach Anspruch 1, das ferner Folgendes umfasst:
Sichern der Kommunikation mit der externen Vorrichtung über den ersten Kommunikationspfad unter Verwendung eines verschlüsselten Schlüsselpaares.

9. Verfahren nach Anspruch 1, wobei der zweite Kommunikationspfad ein induktiver Kommunikationspfad ist und/oder
wobei der zweite Kommunikationspfad ein Nahfeld-Kommunikationspfad ist.

10. Verfahren nach Anspruch 1, wobei der Autorisierungsimpuls Metadaten umfasst, die die Autorisierungsvorrichtung eindeutig identifizieren.

11. System zur Zweifaktoridentifikation, wobei das System eine implantierbare medizinische Vorrichtung (110) und eine Autorisierungsvorrichtung umfasst, wobei:
die implantierbare medizinische Vorrichtung (110) eine Nahbereichsfunkantenne, eine erste Minibereichsantenne, einen Prozessor und einen Speicher umfasst, der Befehle speichert, die, wenn sie durch den Prozessor ausgeführt werden, den Prozessor veranlassen zum:
Empfangen einer Anfrage zur Gerätekopplung von einer externen Vorrichtung über die Nahbereichsfunkantenne;
Senden einer Anfrage für eine Autorisierung über die Nahbereichsfunkantenne an die externe Vorrichtung;
Empfangen eines Autorisierungsimpulses von einer Autorisierungsvorrichtung durch die erste Minibereichsantenne, um die Anfrage zur Gerätekopplung zu autorisieren, wobei die Autorisierungsvorrichtung nicht die externe Vorrichtung ist; und
als Reaktion auf das Empfangen des Autorisierungsimpulses Herstellen der Gerätekopplung der implantierbaren medizinischen Vorrichtung mit der externen Vorrichtung über die Nahbereichsfunkantenne; und
wobei die Autorisierungsvorrichtung einen Autorisierungsimpulsauslöser und eine zweite Minibereichsantenne umfasst, die ausgelegt ist, um mit der ersten Minibereichsantenne zu kommunizieren, wobei der Autorisierungsimpulsauslöser bewirkt, dass ein Autorisierungsimpuls von der zweiten Minibereichsantenne an die erste Minibereichsantenne gesendet wird,
**dadurch gekennzeichnet, dass** die Befehle ferner Befehle umfassen, die, wenn sie durch den Prozessor ausgeführt werden, den Prozessor veranlassen zum:
als Reaktion auf das Empfangen der Anfrage zur Gerätekopplung Aktivieren eines Exklusivitätsfensters; und
Ablehnen aller anderer Anfragen zur Gerätekopplung über die Nahbereichsfunkantenne, während das Exklusivitätsfenster aktiviert ist.

12. System nach Anspruch 11, wobei die erste Minibereichsantenne eine Induktionsspule ist.

13. System nach Anspruch 11, wobei die Autorisierungsvorrichtung eine Ladevorrichtung ist, die dazu in der Lage ist, eine Batterie der implantierbaren medizinischen Vorrichtung zu laden.

14. System nach Anspruch 11, wobei der Autorisierungsimpuls ein verschlüsselter Befehl ist, der die implantierbare medizinische Vorrichtung anweist, die Anfrage zur Gerätekopplung zu autorisieren.

15. System nach Anspruch 11, wobei die Anfrage zur Autorisierung eine Nachricht umfasst, die den Autorisierungsimpuls von der Autorisierungsvorrichtung anfragt.

## Revendications

1. Procédé (300) pour assurer un appariement sécurisé avec un dispositif médical implantable, le procédé comprenant les étapes consistant à :
recevoir (305), par l'intermédiaire du dispositif médical implantable, une demande d'appariement provenant d'un dispositif externe sur une première voie de communication ;
en réponse à la demande d'appariement, transmettre (315), par l'intermédiaire du dispositif médical implantable, un message d'instruction d'autorisation au dispositif externe sur la première voie de communication ;
recevoir (320), par l'intermédiaire du dispositif médical implantable via une seconde voie de communication, une impulsion d'autorisation provenant d'un dispositif d'autorisation pour autoriser la demande d'appariement, dans lequel le dispositif d'autorisation n'est pas le dispositif externe ; et
en réponse à la réception de l'impulsion d'autorisation, finaliser (325), par l'intermédiaire du dispositif médical implantable, l'appariement du dispositif médical implantable avec le dispositif externe,
**caractérisé en ce que** le procédé comprend en outre l'étape consistant à :
en réponse à la réception de la demande d'appariement, activer (310), par l'intermédiaire du dispositif médical implantable, une fenêtre d'exclusivité pour le dispositif externe et refuser toutes les autres demandes d'appariement pendant que la fenêtre d'exclusivité est engagée.

2. Procédé selon la revendication 1, dans lequel le dispositif d'autorisation est un dispositif de charge pour le dispositif médical implantable.

3. Procédé selon la revendication 1, dans lequel l'impulsion d'autorisation est une instruction codée ordonnant au dispositif médical implantable d'autoriser la demande d'appariement.

4. Procédé selon la revendication 1, dans lequel la demande d'appariement est une demande pour initier une relation d'appariement entre le dispositif médical implantable et le dispositif externe en utilisant des ondes radio à ultra-haute fréquence de courte longueur d'onde.

5. Procédé selon la revendication 1, dans lequel le dispositif médical implantable est implanté dans un patient, et dans lequel le dispositif externe est configuré avec une interface pour obtenir des informations à partir du dispositif médical implantable ou pour définir des paramètres de celui-ci.

6. Procédé selon la revendication 1, dans lequel l'engagement de la fenêtre d'exclusivité pour le dispositif externe comprend une annulation d'un message de diffusion, et/ou
dans lequel l'engagement de la fenêtre d'exclusivité comprend un refus de toutes les autres demandes d'appariement sur un protocole de communication de la première voie de communication.

7. Procédé selon la revendication 1, dans lequel le message d'instruction d'autorisation comprend une instruction pour afficher un message sur une interface utilisateur graphique du dispositif externe demandant l'impulsion d'autorisation, et/ou
dans lequel le message d'instruction d'autorisation comprend une instruction pour afficher un message sur une interface utilisateur graphique du dispositif externe demandant l'impulsion d'autorisation avec le dispositif d'autorisation, et/ou
dans lequel le message d'instruction d'autorisation comprend un contenu numérique compréhensible par l'homme décrivant la manière dont un utilisateur peut amener le dispositif d'autorisation à fournir l'impulsion d'autorisation.

8. Procédé selon la revendication 1, comprenant en outre l'étape consistant à :
sécuriser une communication avec le dispositif externe sur la première voie de communication à l'aide d'une paire de clés cryptées.

9. Procédé selon la revendication 1, dans lequel la seconde voie de communication est une voie de communication inductive, et/ou
dans lequel la seconde voie de communication est une voie de communication en champ proche.

10. Procédé selon la revendication 1, dans lequel l'impulsion d'autorisation comprend des métadonnées identifiant de manière unique le dispositif d'autorisation.

11. Système d'autorisation de second facteur, le système comprenant un dispositif médical implantable (110) et un dispositif d'autorisation, dans lequel :
le dispositif médical implantable (110) comprend une antenne radio à courte portée, une première antenne à mini-portée, un processeur et une mémoire stockant des instructions qui, lorsqu'elles sont exécutées par le processeur, amènent le processeur à :
recevoir une demande d'appariement provenant d'un dispositif externe via l'antenne radio à courte portée ;
transmettre une demande d'autorisation au dispositif externe via l'antenne radio à courte portée ;
recevoir une impulsion d'autorisation provenant d'un dispositif d'autorisation via la première antenne à mini-portée pour autoriser la demande d'appariement, dans lequel le dispositif d'autorisation n'est pas le dispositif externe ; et
en réponse à la réception de l'impulsion d'autorisation, réaliser un appariement complet du dispositif médical implantable avec le dispositif externe via l'antenne radio à courte portée ; et
le dispositif d'autorisation comprend un déclencheur d'impulsion d'autorisation et une seconde antenne à mini-portée configurée pour communiquer avec la première antenne à mini-portée, dans lequel le déclencheur d'impulsion d'autorisation provoque la transmission d'une impulsion d'autorisation de la seconde antenne à mini-portée à la première antenne à mini-portée,
**caractérisé en ce que** les instructions comprennent des instructions supplémentaires qui, lorsqu'elles sont exécutées par le processeur, amènent le processeur à :
en réponse à la réception de la demande d'appariement, engager une fenêtre d'exclusivité ; et
refuser toutes les autres demandes d'appariement via l'antenne radio à courte portée pendant que la fenêtre d'exclusivité est engagée.

12. Système selon la revendication 11, dans lequel la première antenne à mini-portée est une bobine d'induction.

13. Système selon la revendication 11, dans lequel le dispositif d'autorisation est un dispositif de charge capable de charger une batterie du dispositif médical implantable.

14. Système selon la revendication 11, dans lequel l'impulsion d'autorisation est une commande codée ordonnant au dispositif médical implantable d'autoriser la demande d'appariement.

15. Système selon la revendication 11, dans lequel la demande d'autorisation comprend un message demandant l'impulsion d'autorisation au dispositif d'autorisation.
